# EUROPEAN PATENT APPLICATION

(11) **EP 1 800 622 A1**
(43) Date of publication of application: **27.06.2007**
(21) Application number: 05405722.9
(22) Date of filing: 23.12.2005
(51) Int. Cl.: A61F 2/16

(54) **Spring-biased injector for an intraocular lens**

(71) Applicant: SDI Surgical Device International GmbH, 2562 Port (CH)
(72) Inventor: Meyer, Rolf, 2562 Port (CH)
(74) Representative: Liebetanz, Michael

(57) **Abstract**

An insertion device for surgical implantation of an intraocular lens in the eye is disclosed. The device comprises a plunger (200) which is displaceable within a sleeve (110) for guided insertion of the lens. The plunger has a hollow portion (212), and a spring (250) is disposed within the hollow portion of the plunger. The spring is elastically compressed when the plunger is advanced in a distal direction, thus creating a rearward biasing force for improved control of the insertion process. In an advantageous embodiment, the spring may be pre-biased, and the spring force acts only in a final phase of advancing the plunger.

## Description

### Field of the Invention

The present invention relates to an insertion device (injector) for surgical implantation of an intraocular lens in the eye.

### Background of the invention

In cataract surgery, an opaque natural lens in a patient's eye is replaced by an artificial intraocular lens (IOL). In this procedure, the natural lens is first removed, usually by phacoemulsification. Then the artificial IOL is inserted. A variety of techniques can be used for the insertion step. Traditionally, the surgeon introduces the lens into the eye with the aid of surgical forceps. Alternatively, a specifically adapted insertion device (injector) may be used. Such a device generally comprises a sleeve and a plunger longitudinally displaceable within the sleeve for advancing an IOL in a deformed (e.g., rolled or folded) state through an opening in a small-diameter nozzle-like portion into the patient's eye, where the lens is then allowed to unfold. The nozzle portion, which is introduced into the eye through a small incision, often has a diameter of no more than 1.5 millimeters.

A variety of different injectors have become known in the art. In one example, the plunger is connected to the housing via a thread. The plunger is advanced longitudinally by rotating a proximal end of the plunger, the thread translating the rotation into a longitudinal displacement. While such a device indeed permits exact guiding of the plunger, the surgeon needs both hands for operating the device, and it is relatively difficult to hold the device straight during operation.

Therefore, syringe-like injectors have been devised which permit one-hand operation. These devices comprise a slidable plunger which can be advanced by simply pushing on a proximal plunger head. In use, the surgeon holds the sleeve between his index finger and middle finger, while he presses the plunger head with the thumb of the same hand.

Correct insertion of an IOL into the eye is a very delicate operation requiring extremely fine control. In particular, it must be avoided that the surgeon "overshoots", whereby the deformed lens would shoot out of the nozzle portion of the insertion device in an uncontrollable manner. For improving control, it has been proposed to provide a well-defined resistance force or rearward bias against the advancement of the plunger.

By the way of example, in US 6,069,791, a coil-type spring is wrapped around a proximal portion of the plunger outside of the housing. However, this is disadvantageous as such an arrangement exposes the spring to the outside environment. The spring can thus easily be damaged, and it can easily be contaminated.

In WO 2005/030097, a spring is provided on a proximal portion of the plunger outside of the housing in an off-center fashion. Upon advancement of the plunger, the spring is compressed against the housing. Again, this spring is exposed to the outside environment and can thus easily be damaged. In addition, the spring can easily be lost.

In US 5,860,984, US 2004/0059343, US 6,251,114 and US 2002/0165610, a coil-type spring is wrapped around one of several different portions of the plunger within the housing. This, however, is disadvantageous since additional space is required for accommodating the spring within the housing, which renders the insertion device bulkier and heavier than necessary.

### Summary of the invention

It is an object of the present invention to provide an insertion device for surgical implantation of an intraocular lens in the eye which provides a rearward bias to the plunger without requiring additional space within the plunger body. It is a further object of the invention to provide an insertion device in which the biasing means are protected from loss or damage.

This and other objects not specifically mentioned are achieved by an insertion device for surgical implantation of an intraocular lens in the eye with the features of claims 1.

Thus, a device is provided which comprises
- a housing comprising a sleeve;
- a plunger manually displaceable within said sleeve in a longitudinal direction, said plunger having a distal tip for advancing said intraocular lens and having a proximal end arranged outside of the sleeve for manually advancing said plunger, said plunger further having a hollow portion;
- a spring disposed within said hollow portion; and
- a first and a second stop, said spring being elastically deformed between said first and second stop along said longitudinal direction when said plunger is advanced in a distal direction from a partially or fully retracted position.

By providing the spring in a hollow portion of the plunger, it is protected from damage. At the same time, no additional space is required within the housing for accommodating the spring, and an improved weight distribution may be achieved. Further advantages are apparent from the following description.

According to a second aspect of the present invention, an insertion device for surgical implantation of an intraocular lens in the eye according to claim 14 is provided. Thus, a device is provided which comprises
- a housing comprising a sleeve;
- a plunger manually displaceable within said sleeve in a longitudinal direction, said plunger having a distal tip for advancing said intraocular lens and having a proximal end arranged outside of the sleeve for manually advancing said plunger,
- a spring; and
- a first stop stationary with said plunger and a second stop longitudinally displaceable relative to said plunger,
wherein said spring is pre-biased between said first and second stop in a fully retracted position of said plunger, wherein said second stop is stationary with said plunger when said plunger is displaced from said fully retracted position to a partially retracted position, said spring thus exerting no biasing force between said housing and said plunger, and wherein said second stop is stationary with said housing when said plunger is advanced in a distal direction beyond said partially retracted position, thus elastically deforming said spring along said longitudinal direction between said first and second stop.

The invention further relates to a corresponding method of inserting an intraocular lens in a patient's eye according to claim 15. Thus, a method is provided that comprises the steps of
- providing an insertion device having a housing with a sleeve and a plunger manually displaceable within said sleeve in a longitudinal direction, said plunger having a distal tip for advancing said intraocular lens and having a proximal end arranged outside of the sleeve for manually advancing said plunger, said device further comprising a spring, a first stop stationary with said plunger and a second stop longitudinally displaceable relative to said plunger, wherein said spring is pre-biased between said first and second stop in a fully retracted position of said plunger, wherein said second stop is stationary with said plunger when said plunger is displaced from said fully retracted position to a partially retracted position, said spring thus exerting no biasing force between said housing and said plunger, and wherein said second stop is stationary with said housing when said plunger is advanced in a distal direction beyond said partially retracted position, thus elastically deforming said spring along said longitudinal direction between said first and second stop;
- manually retracting said plunger into a substantially fully retracted position;
- providing said intraocular lens to said insertion device (e.g., by providing a cartridge containing the lens, or by inserting the lens directly into the suitably designed injector);
- manually advancing said plunger by pressing with a finger, in particular, a thumb, on said proximal end of said plunger, without action of said biasing force of said pre-biased spring into a partially retracted position, thereby advancing said intraocular lens by action of said plunger tip into a nozzle portion connected to said insertion device (where said nozzle portion may, e.g., be formed on a cartridge or on the injector housing itself);
- further advancing said plunger by pressing with the same finger against the action of said biasing force of said pre-biased spring, thereby ejecting said intraocular lens from said nozzle portion.

By providing a biasing force of a pre-biased spring only in the last phase of advancing the plunger when the lens normally is about to leave the device, the surgeon can better determine when that last phase begins, and particularly good control in this last phase is possible. In such a device and method, the spring may be disposed within the plunger, but it is also possible to provide the spring in other locations, e.g., around the plunger within or outside of the housing. The spring is preferably deformed by compression; however, it is also envisaged that the spring is tensioned instead. In the case of a spring that is compressed, pre-bias may be achieved by limiting displacement of the second stop relative to the plunger away from the first stop such that the spring always remains biased. If the spring is instead tensioned, pre-bias may be achieved by limiting movement of the second stop towards the first stop. The second stop will preferably become stationary with the housing by abutting to a retaining element fixedly connected with the housing (e.g., an end cap or some protrusion) when the plunger is displaced beyond the predetermined partially retracted position. The second stop and the corresponding retaining element may be arranged inside or outside of the housing.

In a preferred embodiment, the spring is a coiled (helical, cylindrical) spring. Such springs are reliable, easy to manufacture and well adapted to being disposed in a hollow space like the preferably cylindrical hollow portion within the plunger.

In a preferred embodiment, the hollow portion is a cylindrical bore along the longitudinal plunger axis. The spring is preferably arranged in the hollow portion substantially centrally with respect to the plunger axis. Preferably, at least one slit is provided in a surface of the plunger for accessing the hollow space. In a preferred embodiment, two slits are provided on opposite circumferential sides of the plunger. Preferably, the slits are rectilinear and parallel to the plunger axis, however, they may also be curved.

The first stop is preferably stationary with the plunger. It is preferably arranged near the proximal end of the plunger outside of said housing. The second stop is preferably at least temporarily (i.e., during certain phases of the advancing movement, preferably during a final phase of the advancing movement of the plunger) stationary with said housing when said plunger is moved in the distal direction from the partially or fully retracted position.

In a simple embodiment, the second stop is fixedly connected with the housing, e.g., it may simply take the form of a lug extending into one of the slits in the plunger surface, thus forming a distal spring seating. In a preferred embodiment, however, the second stop comprises a distal stop element being slidably disposed on the plunger, said distal stop element abutting to the housing when the plunger is advanced in the distal direction from a fully or partially retracted position.

Preferably, the distal stop element has a predetermined non-zero distance from the housing when the plunger is in a fully retracted position. In this way, the surgeon does not yet feel any resistance from the spring during an initial phase of advancing the plunger from a fully retracted position to a partially retracted position in which the distal stop element first touches the housing.

Preferably, the hollow portion of the plunger is accessible through at least two parallel longitudinal slits, and the distal stop element comprises a ring slidably arranged around the plunger and a spring seating disposed inside the hollow space. The spring seating then is connected with the ring through at least two of the slits, where the slits are preferably arranged at uniform angular distances on the surface of the plunger. This ensures a high stability and a uniform transmission of the spring force to the stop element, without the danger of canting.

The spring may be pre-biased between the first stop and the second stop when the plunger is in a fully retracted position. In this manner, the surgeon feels a well-defined, non-zero "threshold" resistance when the spring starts to be compressed by advancing the plunger from a fully retracted position or beyond a predetermined partially retracted position.

In order to avoid overshooting of the plunger and damage of the eye by the plunger tip, a limiting structure for limiting the advancing movement of the plunger in the distal direction may be provided. There are many possibilities for constructing such a limiting structure, either inside or outside of the housing, where the limiting structure provides a positive stop to the advancing movement of the plunger. In a preferred embodiment, the limiting structure comprises a pin extending within the hollow portion of the plunger along the longitudinal direction. Preferably, the pin is arranged centrally on the plunger axis. It may be connected either to the first or the second stop, and the other stop then serves as a stop for the pin.

In a preferred embodiment, the proximal end of the plunger is adapted to be manually pressed with a surgeon's finger, in particular, a surgeon's thumb. As a thumb support, the injector of the present invention may comprise a plunger head, preferably of enlarged diameter, mounted on the proximal end of the plunger for manually advancing the plunger. The plunger head may be rotatable relative to the plunger. A finger ring for receiving a surgeon's thumb may be mounted on the plunger head. For preventing uncontrolled rotation of the plunger, the plunger may have a guide groove, and a guide element arranged in said housing may engage in said guide groove,

In a preferred embodiment, which is also useful in injectors without a biasing spring or with a spring arranged in other locations than in a hollow space within the plunger, the guide element comprises a ball engaging in both the guide groove of the plunger and in a second guide groove on an inner surface of the sleeve.

Provisions for easily cleaning the device may be made. In particular, at least one opening may provided in the housing near a proximal end of the housing for introducing a cleaning fluid into the housing, and at least one groove may be provided on a circumferential surface of the housing for attaching a cleaning tool to the device.

### Brief description of the drawings

The invention will be described in more detail in connection with an exemplary embodiment illustrated in the drawings, in which
- Fig. 1: is a perspective view of a preferred embodiment of an injector according to the present invention;
- Fig. 2: is a side elevational view of the injector of Fig. 1;
- Fig. 3: is a top planar view of the injector of Fig. 1;
- Fig. 4: is a cross-sectional view of the injector of Fig. 1 in the plane indicated as 4-4 in Fig. 3;
- Fig. 5: is a detailed cross-sectional view of the rear portion of Fig. 4;
- Fig. 6: is a schematic diagram of force vs. displacement; and
- Fig. 7: is a side elevational view of the rear portion of Fig. 2 together with a cleaning device.

### Detailed description of the Invention

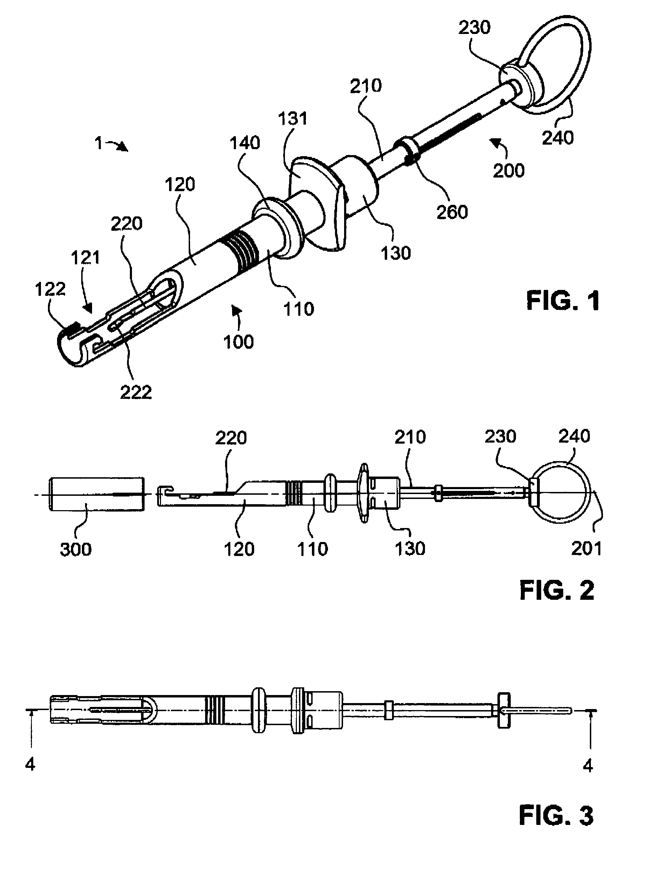

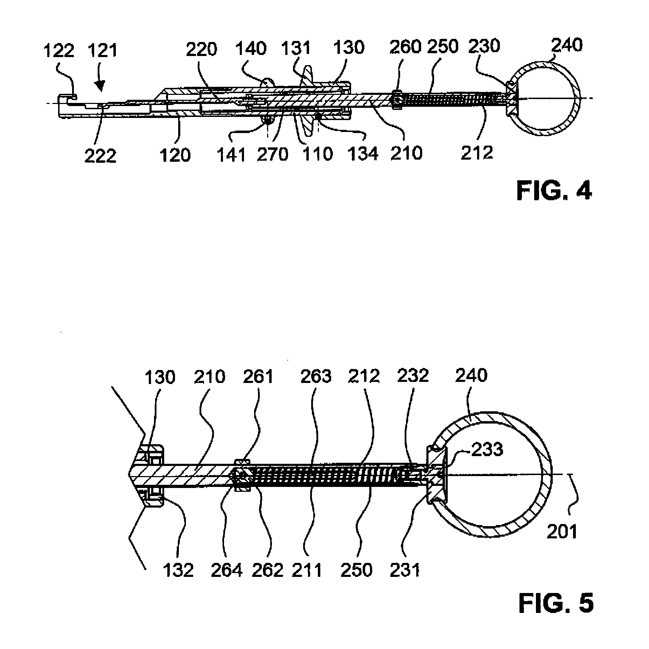

A preferred embodiment of an injector 1 for an intraocular lens according to the present invention is shown in Figs. 1 to 5. The injector comprises a housing generally designated as 100 in which a plunger generally designated as 200 is guided for longitudinal displacement along the plunger axis 201.

The housing comprises a substantially cylindrical sleeve 110 for guiding the plunger, merging into a housing front part 120. In the present embodiment, the housing front part 120 is screwed onto the sleeve 110. Alternatively, it may be connected to the sleeve by other means, or it may be made in one piece with the sleeve. The housing, and in particular the sleeve, is preferably made from a corrosion-resistant and inert metal like titanium. However, it may also be envisaged to be manufactured from a high-strength plastic material.

The housing front part 120 is adapted for receiving a lens cartridge (not shown in the drawings). To this end, the front part 120 has a lateral window 121 and a pair of hooks 122 for holding the lens cartridge in the window 121, such that the plunger tip 222 can enter a longitudinal through bore of the lens cartridge. The lens cartridge has a lens receiving section which will be disposed in the region of the window 121 and a nozzle portion which will extend beyond the front end of the front part 120. An intraocular lens provided in the lens receiving section of the cartridge may be pushed towards the nozzle portion and through an opening therein into a patient's eye by the action of the plunger 200. Depending on the type of lens cartridge, the lens is folded or rolled up during this process, or the lens may be already provided in a rolled-up or folded state in the lens receiving section.

A closure cap 130 is mounted on the rear (proximal) end of the sleeve 110. The closure cap is slid onto the sleeve and fixedly connected to the sleeve with the aid of a small stud screw 134. In an alternative embodiment, the closure cap may be screwed onto the sleeve. A finger support 131 in the form of two radial flanges is formed on the closure cap near its front end. The flanges serve for supporting the index and middle finger when a surgeon grips the injector in a manner like a syringe. Instead of two flanges, a single, ring-like radial flange or any other structure which may serve as a finger support may be provided. Instead of being formed on the cap 130, the finger support may be a separate element, or it may be formed integrally with the sleeve 110.

On the sleeve 110, an optional distance ring 140 is mounted, serving as a front limit stop for the surgeon's fingers. The ring 140 is secured on the sleeve in a releasable manner by a small stud screw 141 and is displaceable along the sleeve after loosening the screw 141 for adapting the distance between the finger support 131 and the ring 140, thereby adjusting the distance to the thickness of a surgeon's fingers. Instead of a ring, any other element having at least one radially extending flange may be provided as a front limit stop, or the front limit stop may be left away completely. In a simplified embodiment, the front limit stop may be integrally formed on the sleeve or may be formed in one piece with the finger support.

The plunger 200 extends longitudinally through the sleeve 110. Its rear (proximal) end portion protrudes from the sleeve 110 through the closure cap 130. The plunger 200 comprises a cylindrical push rod 210 to whose front (distal) end a plunger needle 220 is releasably mounted. However, the plunger may also be formed in one piece. The plunger needle 220 ends in a specifically shaped plunger tip 222 for pushing the lens through the nozzle portion of the cartridge into the patient's eye. At the rear end of the plunger, a plunger head 230 serving as a support for a surgeon's thumb is mounted on the push rod 210. In the present embodiment, the plunger head 230 is mounted on the push rod in a rotatable manner, allowing for rotation of the plunger head 230 around the plunger axis 201. While this is particularly advantageous in specific situations, the plunger head 230 may also be fixedly secured to the push rod 210 instead. An optional finger ring 240 is attached to the plunger head 230 for receiving the surgeon's thumb. This finger ring (handle ring) 240 provides Improved guidance of the plunger 200 in the sleeve 110 and enables a quick release of the plunger 200 into its retracted position after insertion of the lens into the patient's eye. For further details and advantages of the finger ring, reference is made to U.S. patent application publication No, 2004/0097954, the contents of which are incorporated herein by reference in their entirety for teaching an injector having a finger ring.

The plunger 200 is guided in the sleeve 110 with the aid of a ball-bearing assembly 270 disposed in the sleeve 110, i.e., in the rear part of the housing 100. The ball-bearing assembly 270 comprises a bushing for holding a plurality of balls distributed over the length and circumference of the bushing. These balls contact, on the outside of the bushing, the inner wall of the sleeve 110. On the inside of the bushing, the balls contact the outside surface of the push rod 210. In this way, the plunger 200 is guided in the sleeve 110 for longitudinal displacement with very little friction. It should be noted that in this configuration, the ball-bearing assembly 270 itself moves longitudinally in the sleeve 110 when the plunger 200 is displaced. By the way of example, when the plunger is displaced in the sleeve by two centimeters, the ball-bearing assembly is displaced by half this distance, i.e., by one centimeter. Instead, a different type of ball-bearing assembly may be used in which the ball-bearing assembly cannot move longitudinally in the sleeve. In this case, the balls of the ball-bearing assembly will not contact the inner surface of the sleeve. Further details and advantages of providing a ball-bearing assembly in the rear part of the housing are apparent from U.S. patent application publication No. 2003/0040755, the contents of which are incorporated herein by reference in their entirety for teaching the use of a ball bearing in an IOL insertion device. Instead of a ball-bearing assembly, another type of bearing, e.g., a plain (friction) bearing, may be employed.

In the present embodiment, the ball-bearing assembly further comprises a guide ball having a diameter which is larger than that of the bearing balls. A first guide groove is provided in the outer surface of the push rod, while a second guide groove is provided in the inside surface of the sleeve. The guide ball engages in both of these guide grooves, which serve as tracks for the guide ball during longitudinal displacement of the plunger. In this manner, the plunger is prevented from rotation around its axis, without adding any significant resistance force to the advancement of the plunger. In the present embodiment, the guide grooves are rectilinear. However, if it is desired to induce a controlled rotation of the plunger when the plunger is advanced longitudinally, one of both of the guide grooves may be curved, e.g., spiraled. While providing a guide ball in the bearing assembly that is guided in two complementary tracks is advantageous, other means for preventing uncontrolled rotation of the plunger may be employed, e.g., a single guide groove in the surface of the push rod into which engages a guide element, e.g., in the form of a pin or ball, held in the sleeve, where the guide element may be spring-biased, or in the form of a lug formed on the cap, extending into the guide groove.

In order to provide a well-defined rearward force (rearward bias) to the plunger when the same is advanced for insertion of a lens into a patient's eye, a coiled spring 250 is provided. The spring is accommodated in a central, longitudinal bore (hollow space) 212 in a rear portion of the push rod 210. Two rectilinear, longitudinal sifts provide lateral access to the bore 212. With its rear end, the spring abuts to a ferrule 232 screwed into the rear end of the bore. The ferrule 232 thus serves as a proximal stop (rear limit stop) in the form of a rear/proximal spring seating for the spring 250. At the same time, the ferrule 232 serves for receiving the screw 233 with which the plunger head 230 is rotatably mounted on the push rod 210.

At its front end, the spring 250 abuts to a front (distal) spring seating 262. A pin 263 extends towards the rear end along the plunger axis from the spring seating 262 into the inside of the spring, preventing lateral movement of the spring. The front spring seating extends through the two lateral slits to the outside of the push rod 210. It is fixedly connected to a ring 261 surrounding the push rod. The ring 261 and the spring seating 262 together form a front limit stop (distal stop) 260.

For operation of the device, a lens cartridge containing an intraocular lens for implantation is inserted into the cartridge window 121 of the housing front part 120 and secured there by the hooks 122. The surgeon holds the device by gripping the sleeve 110 between his index and middle fingers in a manner that these fingers rest between the finger support 131 and the distance ring 140. The surgeon inserts the nozzle portion of the cartridge into an incision in the patient's eye and advances the plunger until the ring 261 touches the rear end of the closure cap 230. At this point, the surgeon knows that the lens has been forwarded within the cartridge into the nozzle portion and is just about to exit the nozzle portion. When the plunger is now further advanced for ejection of the lens, the ring 261 abuts to the closure cap 230 and remains stationary with the housing 100. In this last phase of advancing the plunger, the spring is compressed, the front spring seating 262 remaining stationary while the push rod and thus the rear spring seating advances further. Thus the spring exerts a rearward force to the plunger which increases with further advancement of the plunger. The plunger may be advanced against the rising biasing force of the spring until finally the rear end of the pin 263 abuts to the ferrule 232 and prevents the plunger from being forwarded any further. In this position, the lens has exited the nozzle portion of the cartridge, and further advancement of the plunger is prevented for avoiding damaging the patient's eye by the plunger tip.

In an advantageous embodiment, the spring is already preloaded (biased) in the fully retracted position of the plunger before being further compressed by advancing the plunger. To this end, the length of the uncompressed spring is chosen larger than the distance available for the spring between the front and rear spring seatings in the configuration of Figs. 1 to 5. When the plunger is advanced beyond the point where the ring 261 first touches the closure cap 230, the resistance force sensed by the surgeon rises suddenly and distinctively by the action of the (preloaded) spring. The surgeon thus knows exactly and intuitively that he has reached the last and most critical phase of the injection procedure where the lens is just about to exit the nozzle portion of the cartridge.

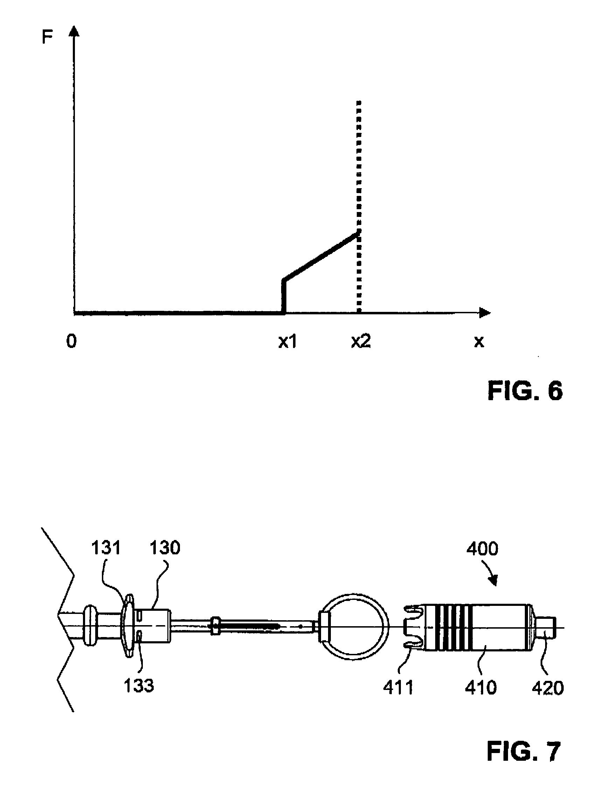

This is illustrated in Fig. 6, which shows a schematic diagram of resistance force F vs. displacement x during advancement of the plunger, where x increases when the plunger is advanced towards the front end of the device. The resistance resulting from the lens being moved in the cartridge is disregarded in Fig. 6, as this resistance depends on the type of lens, the type of cartridge, and it depends in a more complicated, but generally smooth and continuous manner on the displacement. Therefore, Fig. 6 shows the resistance force against an advancement of the plunger when no cartridge is present. When the plunger is advanced in the injector from a fully retracted position (x = 0), there is at first no resistance force, apart from the unavoidable resistance of the bearing. At position x = x1, the plunger has been advanced a partially retracted position where the ring 261 touches the rear of cap 230. When advancing the plunger further, the resistance force suddenly rises to the value given by the amount of preloading (bias) of the spring between seating 262 and ferrule 232. From there, the force rises approximately in a linear manner upon further advancing the plunger, according to Hooke's law F = k x, where the proportionality factor k is the spring constant, which is a characteristic of the spring used. At position x = x2, the pin 263 in the plunger touches the ferrule 232, and the plunger cannot be advanced further. By a proper choice of the spring and of the initial compression of the spring, both the initial resistance force beyond point x = x1 and the slope of the increase of the force with displacement can be easily adjusted to individual needs. Such a force characteristic may also be achieved when the spring is disposed in other locations, e.g., around the plunger, as long as the spring is already preloaded (pre-biased) in the fully retracted position of the plunger and is further compressed only by advancing the plunger beyond a partially retracted position.

The injector according to the embodiment of Figs. 1 to 5 thus provides a number of Particular advantages, such as the following:
- The injector provides a well-defined, controllable resistance force to the advancement of the plunger.
- The resistance force may be chosen to be only applied in the last, most critical phase of advancing the plunger.
- The injector allows for providing a preloaded (pre-biased) spring, thereby allowing the surgeon to intuitively feel that the last phase of advancement has been entered by a sudden rise of resistance.
- The injector provides a well-defined stop for the advancing movement of the plunger.
- As the spring is arranged inside the plunger, it has a smaller size than a spring arranged around the plunger.
- The spring is largely hidden from the surroundings, and can therefore not be damaged easily.
- The spring can easily be replaced after removing the plunger head 230 and the ferrule 232, allowing for easy adaptation of the spring force to individual preferences or easy replacement in case of a failure.
- In practice, the middle finger of the surgeon acts as a support for the weight of the injector, and a symmetrical weight distribution with respect to that support is desirable. The currently proposed arrangement aids in achieving that goal, since the weight of the spring and its seatings acts in a region behind the finger supports 131, and since the total length of the injector may be decreased, thus reducing moments of force (torque), as compared to an injector where the spring is disposed inside the injector body.
- Since the inside space of the sleeve is not needed for accommodating a spring, a sophisticated bearing assembly such as a moving ball-bearing assembly may be employed.

For efficiently cleaning the injector, including the region of the spring, a specifically adapted cleaning device may be used. Such a cleaning device is shown in Fig. 7 together with the rear end of the injector. It comprises a hollow cylindrical body 410. From the front side of the body 410, four resilient click-on protrusions 411 each having a small inwardly extending lug extend along the longitudinal direction. At the rear side, a connector for a tube or a syringe is provided, which allows for a liquid to be introduced into the hollow body. For cleaning the injector, the finger ring 240 is removed from the injector. The cleaning device is then slid over the plunger until the plunger head abuts with the inner rear face of the cleaning device. The cleaning device is then pushed further in the forward direction, thereby advancing the plunger in the sleeve and finally compressing the spring, until the click-on protrusions slide on the circumferential surface of the closure cap 130 and finally get locked with their lugs in four complementary grooves in the circumferential surface of the closure cap 130. An O-ring in the inside of the cleaning device seals the cleaning tool at the circumferential surface of the closure cap 130. A cleaning fluid is then introduced into the cleaning device through connector 420. This cleaning fluid will intimately contact the plunger head, the spring and the surrounding portions of the plunger, thereby removing any dirt and contamination. From the cleaning device, the cleaning fluid enters the inside of the injector through a plurality of small off-axis through-holes 132 in the closure cap 130, the holes extending parallel to the plunger axis. Thus, also the inside of the injector is efficiently cleaned. The cleaning tool is easily removed from the injector by rotating it by an eighth of a turn, thereby releasing the retaining lugs on the click-on protrusions from the grooves 133, and simply pulling it off.

It will be appreciated that the foregoing description refers to a specific preferred embodiment and is to be understood as not limiting the invention. In particular, various modifications are possible without leaving the scope of the present invention. Some examples of such modifications are now described.

In one possible modification, the spring need not be preloaded. The length of the spring and of the slits in the plunger may readily be chosen such that a rearward spring force is applied already during earlier phases of the advancement of the plunger in the injector, including a situation in which a force is already applied in the fully retracted position. It may be desirable in certain situations to have a smoothly rising, continuous bias force when advancing the plunger, which can be easily achieved by these measures.

In a simplified construction, the front spring seating 261 together with the ring 262 may be left away, and instead an element which is fixedly connected to the housing and which extends into one of the slits of the plunger may be employed as a front limit stop (distal stop) for the spring. In the simplest case, such an element may be constituted by a lug formed on the rear end of the closure cap 130 which extends right into the slit. To this end, of course, the slit would have to be made longer than in the embodiment of Figs. 1 to 5 in order to allow the plunger to be pulled out into its fully retracted position.

Instead of a coiled spring, any other type of spring may be used, including cantilever springs or combinations of different spring types. The spring might also be constituted by a piece of resilient material, e.g., rubber etc, which provides an elastic biasing force against the advancement of the plunger.

Instead of a compression spring, a spring exerting a tension force might be employed. In this case, preferably the hollow space of the plunger would extend into the inside of the housing, and the spring would be fixedly connected between a proximal stop which may come into abutment with the housing and a distal stop fixedly connected with the plunger. On pushing the plunger into the housing, the spring would then be tensioned.

### List of reference symbols

- x: Displacement
- x1: First position
- x2: Second (final) position
- F: Force

- 1: Injector
- 100: Housing
- 110: Sleeve
- 120: Front part
- 121: Window for cartridge
- 122: Hook
- 130: Cap
- 131: Finger support
- 132: Opening
- 133: Groove
- 140: Distance ring
- 141: Screw (stud)
- 200: Plunger
- 201: Plunger axis
- 210: Plunger rod
- 211: Longitudinal slit
- 212: Bore
- 220: Plunger needle
- 222: Plunger tip
- 230: Plunger head
- 231: Thumb support
- 232: Ferrule
- 233: Screw
- 240: Finger ring
- 250: Spring
- 260: Distal stop assembly
- 261: Ring
- 262: Spring seating
- 263: Pin
- 270: Ball bearing assembly
- 300: Protective cap
- 400: Cleaning tool
- 410: Cleaning tool body
- 411: Click-on protrusion
- 420: Connector

## Claims

1. An insertion device (1) for surgical implantation of an intraocular lens in the eye, said device comprising
- a housing (100) comprising a sleeve (110);
- a plunger (200) manually displaceable within said sleeve (110) in a longitudinal direction, said plunger (200) having a distal tip (222) for advancing said intraocular lens and having a proximal end (230) arranged outside of the sleeve (110) for manually advancing said plunger (200)
**characterized in that** said plunger (200) has a hollow portion (212), a spring (250) being disposed within said hollow portion (212), and that the insertion device (1) comprises a first stop (232) and a second stop (260), said spring (250) being elastically compressed between said first and second stop along said longitudinal direction when said plunger (200) is advanced in a distal direction from a partially or fully retracted position.

2. The device of claim 1, wherein said spring (250) is a coiled spring.

3. The device of claim 1, wherein said plunger (200) defines a plunger axis, and wherein said spring (250) is arranged in said hollow portion substantially centrally with respect to said plunger axis.

4. The device of claim 1, wherein at least one slit (211) is provided in a surface of said plunger for accessing said hollow space.

5. The device of claim 1, wherein said first stop (232) is stationary with said plunger and arranged near said proximal end of said plunger outside of said housing.

6. The device of claim 1, wherein said second stop (260) is at least temporarily stationary with said housing (100) when said plunger (200) is moved in said distal direction from said partially or fully retracted position.

7. The device of claim 1, wherein said second stop (260) is fixedly connected with said housing (100).

8. The device of claim 1, wherein said second stop (260) comprises a distal stop element (261, 262) being slidably disposed on said plunger (200), said distal stop element (261, 262) abutting to said housing (100) when said plunger (200) is advanced from a partially or fully retracted position.

9. The device of claim 8, wherein said distal stop element (261, 262) has a predetermined non-zero distance from said housing (100) when said plunger is in a fully retracted position.

10. The device of claim 8, wherein said hollow portion (212) is accessible through at least two parallel longitudinal slits (211), and wherein said distal stop element (261, 262) comprises a ring (261) slidably disposed around said plunger (200) and a spring seating (262) disposed inside said hollow space (212), said spring seating (262) being connected with said ring (261) through at least two of said slits (211).

11. The device of claim 1, wherein said spring (250) is pre-biased between said first stop (232) and said second stop (262) when said plunger (200) is in a fully retracted position.

12. The device of claim 1, comprising a limiting structure (263, 232) for limiting an advancing movement of said plunger (200) in said distal direction.

13. The device of claim 12, wherein said limiting structure (263, 232) comprises a pin (263) extending within said hollow portion (212) in said longitudinal direction.

14. An insertion device for surgical implantation of an intraocular lens in the eye, said device comprising
- a housing (100) comprising a sleeve (110);
- a plunger (200) displaceable within said sleeve (110) in a longitudinal direction, said plunger (200) having a distal tip (222) for advancing said intraocular lens and having a proximal end (230) arranged outside of the sleeve (110) for manually advancing said plunger (200); and
- a spring (250),
**characterized in that** the device comprises a first stop (232) stationary with said plunger (200) and a second stop (260) longitudinally displaceable relative to said plunger (200), that said spring (250) is pre-biased between said first and second stop in a fully retracted position of said plunger (200), that said second stop (260) is stationary with said plunger (200) when said plunger (200) is displaced from said fully retracted position to a partially retracted position, said spring (250) thus exerting no biasing force between said housing (100) and said plunger (200), and that said second stop (260) is stationary with said housing (100) when said plunger (200) is advanced in a distal direction beyond said partially retracted position, thus elastically compressing said spring (250) along said longitudinal direction between said first and second stop.

15. A method of inserting an intraocular lens in a patient's eye, said method comprising the steps of
- providing an insertion device (1) having a housing (100) with a sleeve (110) and a plunger (200) displaceable within said sleeve (110) in a longitudinal direction, said plunger (200) having a distal tip (222) for advancing said intraocular lens and having a proximal end (230) arranged outside of the sleeve (110) for manually advancing said plunger (200), said device (1) further comprising a spring (250), a first stop (232) stationary with said plunger (200) and a second stop (260) longitudinally displaceable relative to said plunger (200), wherein said spring (250) is pre-biased between said first and second stop in a fully retracted position of said plunger (200), wherein said second stop (260) is stationary with said plunger (200) when said plunger (200) is displaced from said fully retracted position to a partially retracted position, said spring (250) thus exerting no biasing force between said housing (100) and said plunger (200), and wherein said second stop (260) is stationary with said housing (100) when said plunger (200) is advanced in a distal direction beyond said partially retracted position, thus elastically compressing said spring (250) along said longitudinal direction between said first and second stop;
- retracting said plunger (200) into a substantially fully retracted position;
- providing said intraocular lens to said insertion device (1);
- advancing said plunger (200) without action of said biasing force of said pre-biased spring (250) into said partially retracted position, thereby advancing said intraocular lens by action of said plunger tip (222) into a nozzle portion connected to said insertion device (1);
- further advancing said plunger (200) against the action of said biasing force of said pre-biased spring (250), thereby ejecting said intraocular lens from said nozzle portion.
